# EUROPEAN PATENT APPLICATION

(11) **EP 3 909 646 A1**
(43) Date of publication of application: **17.11.2021**
(21) Application number: 20175042.9
(22) Date of filing: 15.05.2020
(51) Int. Cl.: A61P 31/14, A61K 38/04

(54) **BRADYKININ B2 RECEPTOR ANTAGONIST FOR TREATMENT OF SARS-COV2-INFECTION**

(71) Applicant: Helmholtz Zentrum München Deutsches Forschungszentrum für Gesundheit und Umwelt (GmbH), 85764 Neuherberg (DE); Klinikum rechts der Isar der Technischen Universität München, 81675 München (DE)
(72) Inventor: Chaker, Adam, 81927 München (DE); Schmidt-Weber, Carsten, 80939 München (DE); Jakwerth, Constanze, 80689 München (DE); Zissler, Ulrich, 83376 Truchtlaching (DE); Protzer, Ulrike, 81735 München (DE); Feuerherd, Martin, 85774 Unterföhring (DE)
(74) Representative: Engelhard, Markus

(57) **Abstract**

The present invention relates to a bradykinin B2 receptor (BKB2R)-antagonist for use in a method of preventing progression of SARS-CoV2-infection in a patient, or for use in a method of treating a SARS-CoV2-infection in a patient. Furthermore, the present invention relates to pharmaceutical compositions comprising a BKB2R-antagonist and to their uses.

## Description

The present invention relates to a bradykinin B2 receptor (BKB2R)-antagonist for use in a method of preventing progression of SARS-CoV2-infection in a patient, or for use in a method of treating a SARS-CoV2-infection in a patient. Furthermore, the present invention relates to pharmaceutical compositions comprising a BKB2R-antagonist and to their uses.

Since the outbreak of severe acute respiratory syndrome (SARS) two decades ago in Asia, a considerable number of SARS-related corona viruses have been identified in bats as their natural reservoir hosts. It has been surmised that bats' fierce immune response to viruses can drive viruses to replicate faster, as result of which their effects on other species, upon transmission to such other species, may cause havoc especially if such other species show less fierce immune responses to viruses. In early January 2020, the identification and characterization of a new corona virus was reported which caused an epidemic of acute respiratory syndrome in humans in Wuhan, in China. The full-length genome sequence was obtained from viruses isolated from patients at the early stage of the outbreak. The sequences are almost identical and share 79.6% sequence identity to the SARS-CoV, i.e. the causative agent of SARS. Furthermore, the new corona virus has a 96% identity at the whole-genome level to a bat corona virus. The new virus identified in early January 2020 was termed SARS-CoV2. In contrast to SARS-CoV, the new virus SARS-CoV2 seems to be much more able to transmit from human to human, thus making it a highly contagious agent. Because in approximately 15% of all infection cases, severe courses of disease may be observed requiring special treatment, often times involving intensive care in hospitals, and because the virus has meanwhile spread to more than 200 countries worldwide, this is now a pandemic causing serious problems to the global economy and placing a heavy burden on national health systems on a global scale.

Many patients infected with SARS-CoV2 will initially, in a first phase, experience mild to moderate respiratory illness only and recover without requiring special treatment. Patients typically and primarily present with mild symptoms, such as mild throat pain, loss of olfaction, and other common flu-like symptoms. However, sometimes the disease may progress to a second phase, particularly in older people and people with underlying medical problems, such as cardiovascular disease, chronic respiratory disease, cancer and obesity. In these cases, patients are more likely to develop serious illness. In such more serious cases, the infection may deteriorate into pneumonia and may culminate in acute respiratory distress syndrome (ARDS), sometimes accompanied by concomitant cytokine storm syndrome, sepsis, and/or multi-organ failure. Symptoms often observed in more severe cases are lung edema, dysregulation of vasculature, and alveolar collapse along with a massive release of mainly inflammatory cytokines ("cytokine storm"). The cytokine storm goes along with a systemic release of multiple cytokines including IL-6, IL-8, IL-10, TNF, CCL-2, CCL-3, CCL-5, CXCL10 and IL-1α, indicating possibly the need for immunosuppressive drugs. The use of corticosteroids is, however, controversially discussed as a mechanistic knowledge on the influence of immunosuppressant is not available. The prevention of the second phase of a SARS-CoV2-infection is therefore of a particular interest for the prevention of a fatal disease escalation.

Currently, no specific medication is officially recommended, and no cure is available for the disease caused by a SARS-CoV2-infection. Despite frantic efforts by the global pharmaceutical community, there is no vaccine available yet. The US FDA recently granted permission for some medications, authorized for other diseases, to be used to treat severe SARS-CoV2-infections when no other options are available. Two malaria drugs - hydroxychloroquine and chloroquine - and an antiviral drug, Remdesivir, have been approved for this use by the FDA. Generally, treatment is directed a relieving symptoms and may include the use of pain relievers, such as ibuprofen or acetaminophen, cough syrup or medication, rest and fluid intake. In more severe cases, none-invasive oxygen therapy is used to alleviate subnormal oxygen levels in the blood. Critical cases are treated by using prone positioning ventilation and nitric oxide inhalation therapy alone or together with venous extracorporeal membrane oxygenation (ECMO). However, successful treatment is far from guaranteed and appears to depend on many factors, including the availability of intensive care units (ICUs) in hospitals, oxygen therapy, ventilation and extracorporeal membrane oxygenation places. Even in countries with a developed healthcare system, these are limited.

Thus, there remains a need in the art for alternative treatment options.

In a first aspect, the present invention relate to a bradykinin B2 (BKB2R)-antagonist for use
a) in a method of preventing a SARS-CoV2-infection in a patient to progress to a disease stage characterized by at least one or several of acute respiratory distress syndrome (ARDS), systemic inflammatory response syndrome, severe inflammation of the lower respiratory airways, alveolar collapse and viral sepsis;
   or
b) in a method of treating a SARS-CoV2-infection in a patient;
wherein said method a) or b) involves exposing epithelial tissue of a patient having or suspected of having a SARS-CoV2-infection, to said bradykinin B2 receptor (BKB2R)-antagonist.

In one embodiment, said epithelial tissue is characterized by expressing one or several viral entry proteins, preferably selected from angiotensin converting enzyme 2 (ACE2) and transmembrane protease, serine (TMPRSS) family members, such as transmembrane protease, serine 2 (TMPRSS2), wherein, more preferably, said epithelial tissue expresses both angiotensin converting enzyme 2 (ACE2) and transmembrane protease, serine 2 (TMPRSS2).

In one embodiment, said BKB2R-antagonist exerts a protective effect on epithelial cells in said epithelial tissue against SARS-CoV2-infection.

In particular, said protective effect of said BKB2R-antagonist on epithelial cells is achieved by said BKB2R-antagonist reducing or interfering with SARS-CoV2-entry into epithelial cells.

In one embodiment, reducing or interfering with SARS-CoV2-entry into the epithelial cells is achieved by said BKB2R-antagonist reducing expression of angiotensin converting enzyme 2 (ACE2), alone or in combination with reducing expression of transmembrane protease, serine 2 (TMPRSS2), in particular IFN-gamma-induced expression of transmembrane protease, serine 2 (TMPRSS2). Typically, the expression of ACE2 and/or TMPRSS2 in said epithelial cells is reduced by said BKB2R-antagonist.

In one embodiment, said epithelial tissue of a patient is epithelial tissue of the respiratory tract and/or of the gastrointestinal tract. In one embodiment, said epithelial tissue is epithelial tissue primed by inflammatory conditions, such as co-existing viral or bacterial infections or interferon-driven inflammation. In one embodiment, said epithelial tissue primed by inflammatory conditions is epithelial tissue of the respiratory tract and/or of the gastrointestinal tract, preferably of the respiratory tract.

In one embodiment, said epithelial tissue is
a) epithelial tissue of the respiratory tract and includes one or several of:
   mouth epithelial tissue, nasal epithelial tissue, sinus epithelial tissue, pharyngeal epithelial tissue, laryngeal epithelial tissue, tonsillar epithelial tissue, tracheal epithelial tissue, bronchial epithelial tissue, pulmonary epithelial tissue and alveolar epithelial tissue;
   or
b) epithelial tissue of the gastrointestinal tract and includes one or several of:
   epithelial tissue of the mouth, epithelial tissue of the pharynx, epithelial tissue of the esophagus, epithelial tissue of the stomach, epithelial tissue of the duodenum, epithelial tissue of the jejunum, epithelial tissue of the ileum, epithelial tissue of the appendix vermiformis, epithelial tissue of the cecum, epithelial tissue of the colon, epithelial tissue of the rectum, and epithelial tissue of the anus.

In one embodiment, said epithelial tissue of a patient is
a) epithelial tissue of the respiratory tract and includes one or several types of epithelial cells of the upper respiratory tract, such as basal, ciliated, tuft or goblet cells; epithelial cells of the lower respiratory tract, such as basal, ciliated, goblet, clara cells, type-i- and -2 pneumocytes; and/or tonsillar epithelial cells;
   or
b) epithelial tissue of the gastrointestinal tract and includes one or several types of epithelial cells the gastrointestinal tract, such as enterocytes, M-cells, goblet cells and tuft cells.

In one embodiment, said BKB2R-antagonist is a selective BKB2R-antagonist, preferably selected from icatibant, B-9972, PHA-022121, FR173657, Fasitibant, and antibodies, or antibody fragments, against BKB2R.

In one embodiment, exposing epithelial tissue of a patient having or suspected of having a SARS-CoV2-infection, to said bradykinin B2 receptor (BKB2R)-antagonist, occurs by administering said BKB2R-antagonist to said patient.

In one embodiment, in said use, said BKB2R-antagonist is administered to said patient at one or several stages selected from:
- early asymptomatic disease stage;
- early mild or moderate disease stage; or
- within a period of 1-10 days, preferably 1-5 days, after onset of symptomatic SARS-CoV2-infection.

In one embodiment, said administration is by systemical administration or local administration, wherein preferably said systemical administration is by injection, more preferably subcutaneous injection; and wherein preferably said local administration is by topical application or inhalative administration.

In one embodiment, said local administration is by inhalative administration, preferably by aerosol administration or spray administration; or wherein said local administration is by topical administration, preferably by administration of a gel, administration of a liposomal fluid, administration of an aerosol, administration of a spray, and/or administration of drops, wherein, more preferably, said topical administration is a nasal administration, an oral administration or a conjunctival administration.

In one embodiment, during said use, said BKB2R-antagonist is administered at a dosage of 1 - 100mg daily, preferably 1 - 60mg daily, more preferably 1 - 30 mg daily; for a duration of up to 21 consecutive days.

In one embodiment, said method is performed in order to exert a protective effect on epithelial cells in said epithelial tissue against SARS-CoV2-infection, preferably without compromising an antiviral activity mounted by the patient's immune system, in particular an antiviral activity mounted in said epithelial cells.

In one embodiment, said method results in a reduction of virus titer.

In a further aspect, the present invention also relates to a pharmaceutical composition comprising a BKB2R-antagonist, said composition being configured to allow administration of said BKB2R-antagonist by way of inhalation or topical application; wherein, preferably, said pharmaceutical composition is formulated as one of the following:
- an aerosol comprising said BKB2R-antagonist, or a composition configured to be able to form such aerosol,
- a gel comprising said BKB2R-antagonist,
- a microcapsule formulation comprising microencapsulated BKB2R-antagonist,
- a liposomal formulation comprising liposomes containing said BKB2R-antagonist,
- a spray formulation comprising said BKB2R-antagonist, or
- a drop formulation comprising said BKB2R-antagonist.

The BKB2R-antagonist, comprised in a pharmaceutical composition in accordance with this aspect of the present invention, is as defined further above.

In a further aspect, the present invention also relates to a pharmaceutical composition comprising a BKB2R-antagonist, as defined herein, for use
a) in a method of preventing a SARS-CoV2-infection in a patient to progress to a disease stage characterized by at least one or several of acute respiratory distress syndrome (ARDS), systemic inflammatory response syndrome, severe inflammation of the lower respiratory airways, alveolar collapse and viral sepsis;
   or
b) in a method of treating a SARS-CoV2-infection in a patient;
   wherein said method a) or b) involves exposing epithelial tissue of a patient having or suspected of having a SARS-CoV2-infection, to said bradykinin B2 receptor (BKB2R)-antagonist.

According to this aspect, the epithelial tissue and the BKB2R-antagonist is as defined herein.

In a further aspect, the present invention also relates to the use of a bradykinin B2 receptor (BKB2R)-antagonist in the manufacture of a medicament for
a) preventing a SARS-CoV2-infection in a patient to progress to a disease stage characterized by at least one or several of acute respiratory distress syndrome (ARDS), systemic inflammatory response syndrome, severe inflammation of the lower respiratory airways, alveolar collapse and viral sepsis;
   or
b) treating a SARS-CoV2-infection in a patient;
   wherein said preventing a) or said treating b) involves exposing epithelial tissue of a patient having or suspected of having a SARS-CoV2-infection, to said bradykinin B2 receptor (BKB2R)-antagonist.

Again, the BKB2R-antagonist, the epithelial tissue of a patient are as defined herein.

In a further aspect, the present invention also relates to a method of preventing a SARS-CoV2-invention in a patient to progress to a disease stage characterized by at least one or several of acute respiratory distress syndrome (ARDS), systemic inflammatory response syndrome, severe inflammation of the lower respiratory airways, alveolar collapse and viral sepsis or to a method of treating a SARS-CoV2-infection in a patient;
wherein said method a) or b) involves exposing epithelial tissue of a patient having or suspected of having a SARS-CoV2-infection, to said bradykinin B2 receptor (BKB2R)-antagonist.

The present inventors have surprisingly found that bradykinin B2 receptor (BKB2R)-antagonists have a broad suppressive effect on gene expression in epithelial cells, and in particular on the expression of angiotensin converting enzyme 2 (ACE2). This pertains to both the transcriptional level and the protein level. Moreover, the inventors also found that BKB2R-antagonists significantly reduced expression of transmembrane protease, serine 2 (TMPRSS2). Angiotensin converting enzyme 2 (ACE2) had previously already been reported to act as an entry receptor of SARS-CoV2. TMPRSS2 has previously been reported to activate SARS-CoV2 by "priming" the spike protein of the virus (Hofmann et al., Cell, 2020, 181(2); pp. 271-280). Moreover, the present inventors found that upon infection with SARS-CoV2, epithelial cells show an increase in ACE2 and TMPRSS2 expression, which increase can be reversed, or reduced/avoided in the first place, by exposure of epithelial cells to BKB2R-antagonists. This reversal or reduction of expression manifests itself subsequently in a reduction of virus titer. Without wishing to be bound by any theory, the present inventors believe that BKB2R-antagonists exert a protective effect on epithelial cells by downregulating the respective viral entry proteins, most notably ACE2 and TMPRSS2. This protective effect is herein also sometimes referred to as a cytoprotective effect. Such cytoprotective effect on epithelial cells may manifest itself in a prevention of viral infection (e.g. by SARS-CoV2) in the first place, or in a reduction of viral spread, after an initial infection has taken place.

Accordingly, the present inventors envisage a bradykinin B2 receptor-antagonist for use
a) in a method of preventing a SARS-CoV2-infection in a patient to progress to a disease stage characterized by at least one or several of acute respiratory distress syndrome (ARDS), systemic inflammatory response syndrome, severe inflammation of the lower respiratory airways, alveolar collapse and viral sepsis;
   or
b) in a method of treating a SARS-CoV2-infection in a patient;
   wherein either method a) or b) involves the exposure of epithelial tissue of a patient having or suspected of having a SARS-CoV2-infection, to said bradykinin B2 receptor (BKB2R)-antagonist.

The term "acute respiratory distress syndrome" as used herein, refers to a respiratory failure, symptoms of which may include shortness of breath, rapid breathing, low oxygenation of the blood and bluish skin coloration. In many instances, such ARDS is also associated with or, accompanied by a widespread inflammation of the lungs.

The term "systemic inflammatory response syndrome" (SIRS), as used herein, refers to a condition of the body involving inflammation, organ dysfunction and organ failure. In many instances, it is also accompanied by a massive release of cytokines, mainly inflammatory cytokines.

As used herein, the term "systemic inflammatory response syndrome", is sometimes also used synonymously with "cytokine storm" or "cytokine storm syndrome".

In typical embodiments of the above mentioned methods a) or b), such methods involve an exposure of epithelial tissue of a patient to bradykinin B2-receptor-antagonists (BKB2R-antagonists). Typically, the patient is one that has or is suspected of having a SARS-CoV2-infection. In one embodiment, the patient is a mammal, preferably a human being.

In one embodiment, the epithelial tissue of the patient is characterized by expressing one or several viral entry proteins. Preferred examples of such viral entry proteins are the angiotensin converting enzyme 2 (ACE2) and family members of the transmembrane protease, serine (TMPRSS) family. A particularly preferred example is the transmembrane protease, serine 2 (TMPRSS2). In one embodiment, the epithelial tissue of the patient expresses both angiotensin converting enzyme 2 (ACE2) and transmembrane protease, serine 2 (TMPRSS2).
In one embodiment, the BKB2R-antagonist exerts the protective effect on epithelial cells in the epithelial tissue against a SARS-CoV2-infection. Without wishing to be bound by any theory, the inventors believe that the protective effect exerted by the BKB2R-antagonist on epithelial cells is achieved by such antagonist reducing or interfering with an entry of SARS-CoV2 into epithelial cells. More particularly, again, without wishing to be bound by any theory, the present inventors believe that a reduction or interference of the BKB2R-antagonist with the entry of SARS-CoV2 into the epithelial cells is achieved by the fact that BKB2R-antagonist reduces the expression of angiotensin converting enzyme 2 (ACE2), alone or in combination with reducing the expression of transmembrane protease, serine 2 (TMPRSS2). More particularly, and in a preferred embodiment, with respect to TMPRSS2, it is an IFN-γ-induced of expression of TMPRSS2, which is reduced by the BKB2R-antagonist. Such interferon-γ-induced expression of transmembrane protease, serine 2 is particularly observed in instances where interferon-gamma has been induced as a result of an infection, e. g. a viral infection, in particular a SARS-CoV2-infection. The present inventors have surprisingly found that, contrary to earlier findings by others, a SARS-CoV2-infection appears to increase the expression of both ACE2 and TMPRSS2. According to the findings of the present inventors, such increase in expression of ACE2 and TMPRSS2 can be reversed or prevented/reduced, if the respective epithelial tissue is exposed to a BKB2R-antagonist. This opens up a wide range of treatment options that were hitherto not available. For example, such exposure of epithelial tissue to a BKB2R-antagonist may occur as a preventive measure, aimed at avoiding an infection of epithelial tissue in the first place, or aimed at least at reducing the degree thereof. Hence, for example, in these instances, such BKB2R-antagonist may be administered prophylactically to a patient, when it is not even clear yet whether the patient suffers from a SARS-CoV2-infection at all. In another embodiment, such BKB2R-antagonist may be administered to a patient when such patient has been diagnosed as being SARS-CoV2 positive, but does not show any symptoms or only mild to moderate symptoms. In the latter case, it is particularly desirable to prevent the patient from going into a second phase of disease with severe symptoms and requiring special care.

In one embodiment, the epithelial tissue that is exposed to said BKB2R-antagonist is epithelial tissue of the respiratory tract of said patient. In another embodiment, the epithelial tissue exposed to said BKB2R-antagonist is epithelial tissue of the gastrointestinal tract of said patient. In yet another embodiment, the epithelial tissue that is being exposed to said BKB2R-antagonist is epithelial tissue of the respiratory tract and epithelial tissue of the gastrointestinal tract.

In one embodiment, the epithelial tissue that is exposed to said BKB2R-antagonist is an epithelial tissue that has been primed by inflammatory conditions. Such priming by inflammatory conditions may for example have occurred by a previous viral infection or bacterial infection that may have occurred prior to exposure of the epithelial tissue to the BKB2R-antagonist. The epithelial tissue that has been primed by an inflammatory condition may have been primed for example by an interferon-driven inflammation, wherein, such interferon-driven inflammation, in turn, may have been caused by a viral infection or bacterial infection. Such viral infection or bacterial infection may have occurred prior to exposure of the epithelial tissue to the BKB2R-antagonist, or it may be a viral or bacterial infection that is co-existing or happens concomitantly to exposure of the epithelial tissue to the BKB2R-antagonist.

In one embodiment, the epithelial tissue which is exposed to a bradykinin B2 receptor (BKB2R)-antagonist is epithelial tissue of the respiratory tract and/or of the gastrointestinal tract. In one embodiment, only epithelial tissue of the respiratory tract is exposed to said BKB2R-antagonist. In another embodiment, only epithelial tissue of the gastrointestinal tract is exposed to said BKB2R-antagonist. In yet another embodiment, epithelial tissue of both the respiratory tract and of the gastrointestinal tract are exposed to said BKB2R-antagonist. The choice which epithelial tissue is exposed to said BKB2R-antagonist may be steered by the way of administration and/or the chosen pharmaceutical formulation.

In one embodiment, the epithelial tissue of the respiratory tract includes one or several of: mouth epithelial tissue, nasal epithelial tissue, sinus epithelial tissue, pharyngeal epithelial tissue, laryngeal epithelial tissue, tonsillar epithelial tissue, tracheal epithelial tissue, bronchial epithelial tissue, pulmonary epithelial tissue and alveolar epithelial tissue. In one embodiment, the epithelial tissue of the respiratory tract is airway epithelial tissue and, preferably, includes, in particular one or several of mouth epithelial tissue, nasal epithelial tissue, pharyngeal epithelial tissue, laryngeal epithelial tissue, tracheal epithelial tissue, bronchial epithelial tissue, pulmonary epithelial tissue and alveolar epithelial tissue.

In embodiments, wherein the epithelial tissue is epithelial tissue of the respiratory tract, the present inventors envisage a preferred exposure of such epithelial tissue to said BKB2R-antagonist. Such a preferred exposure may, for example, be achieved by targeted administration of said BKB2R-antagonist to said tissue. Examples thereof include an administration by way of inhalation or by local administration, such as by means of a spray.

The term "exposing epithelial tissue of a patient to a bradykinin B2 receptor (BKB2R)-antagonist", as used herein, is meant to refer to a scenario in which said patient is administered a BKB2R-antagonist in such a manner that the epithelial tissue comes into contact with said BKB2R-antagonist. In one embodiment, exposing epithelial tissue of a patient to said BKB2R-antagonist implies that said BKB2R-antagonist may interact with said epithelial tissue, e. g. if said epithelial tissue is exposed to a BKB2R-receptor, said antagonist may interact with said BKB2R-receptor on exposure of said epithelial tissue to said antagonist.

In one embodiment, the epithelial tissue of the gastrointestinal tract includes one or several of: epithelial tissue of the mouth, epithelial tissue of the pharynx, epithelial tissue of the esophagus, epithelial tissue of the stomach, epithelial tissue of the intestines. In particular, such epithelial tissue of the intestines may include one or several epithelial tissues of the duodenum, jejunum, ileum, appendix vermiformis, epithelial tissue of the cecum, epithelial tissue of the colon, of the rectum, and/or of the anus.

In one embodiment, the epithelial tissue may be characterized by including one or several type(s) of epithelial cells. For example, in one embodiment, the epithelial tissue is epithelial tissue of the respiratory tract and includes one or several types of epithelial cells of the upper respiratory tract, such as basal cells, ciliated cells, tuft cells or goblet cells; it may also, additionally or instead include epithelial cells of the lower respiratory tract, such as basal cells, ciliated cells, goblet, clara cells, type-i- and -2 pneumocytes; and/or tonsillar epithelial cells.

In yet another embodiment, the epithelial tissue may be epithelial tissue of the gastrointestinal tract and may includes one or several types of epithelial cells of such gastrointestinal tract, in particular enterocytes, M-cells, goblet cells and tuft cells.

In one embodiment, the BKB2R-antagonist to be used in the context of the present invention is selected from icatibant, B-9972, PHA-022121, FR173657, Fasitibant and antibodies, or antibody fragments, against BKB2R. For example, some BKB2R-antagonists are peptidic or peptidomimetic in nature, in that they are peptides comprising amino acids and, possibly, amino acid analogues or derivatives or non-natural amino acids. In one embodiment, the BKB2R-antagonist is a selective BKB2R-antagonist. In one embodiment, the term "selective BKB2R-antagonist", as used herein refers to an antagonist, which preferentially antagonises bradykinin B2 receptor (BKB2R) over bradykinin B1 receptor (BKB1R). In one embodiment, the term "BKB2R-antagonist" may refer to the free base, but also to pharmaceutically acceptable salts, if such salts are chemically feasible. Pharmaceutically acceptable salts may include acid addition salts, such as are formed from acids which form non-toxic acid anions such as the hydrochloride, hydrobromide, sulphate, phosphate or acid phosphate, acetate, maleate, fumarate, lactate, tartrate, citrate and gluconate salts.

In one embodiment, the BKB2R-antagonist is icatibant or a pharmaceutically acceptable salt thereof. An example of a pharmaceutically acceptable salt of icatibant is icatibant acetate. Currently, icatibant is authorised and marketed to be used for the symptomatic treatment of hereditary angioedema (HAE). HAE is caused by an absence or dysfunction of C1-esterase-inhibitor. HAE attacks are accompanied by an increased release of bradykinin which is the key mediator in the development of clinical symptoms of HAE. Icatibant is a selective competitive antagonist at the bradykinin type 2 receptor (herein also referred to as "bradykinin B2 receptor-antagonist" or "BKB2R-antagonist") (Charignon D, et al. Expert Opin Pharmacother. 2012;13(15):2233-47). Icatibant is a synthetic decapeptide with a structure similar to bradykinin, but having five non-proteinogenic amino acids.

It is thus a peptidomimetic and, in one embodiment, has the structural formula

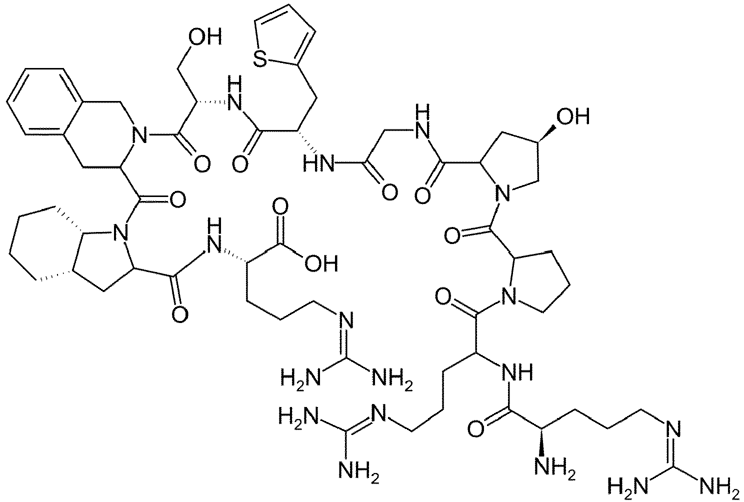

B-9972 as BKB2R-antagonist has been described in Stuart et al. Peptides, 2005, 26(8); pp. 1292-1300. PHA-022121 has been described by Lesage et al., Journal of Allergy and Clinical Immunology, Vol. 145, 2020, Abstract 346. FR173657 has been described by Asano et al. 1997, Br.J.Pharmacol., 120; pp. 617-624. Fasitibant, also known as MEN-16132 has been described by Cucchi et al. 2005, European Journal of Pharmacology, 528; pp. 7-16.

The present inventors' finding that BKB2R-antagonists exert a protective effect on epithelial cells against SARS-CoV2-infection, as disclosed herein, is entirely surprising and was not to be expected in view of their known function as competitors of bradykinin at the BKB2R and their authorised use for the treatment of HAE. The present inventors' findings thus open up a whole new area of treatment options hitherto not available.

In one embodiment, the exposure of epithelial tissue of a patient having or suspected of having a SARS-CoV2 infection to a BKB2R-antagonist occurs by administering the BKB2R-antagonist to the patient. In one embodiment, said administration occurs prophylactic, that is, for example, in a patient suspected of having a SARS-CoV2-infection, but not showing any relevant symptoms (yet), and/or in a patient who belongs to a high risk group, such as a patient suffering from other underlying health conditions, or a smoker or a patient whose lung may be already be affected by other conditions, such as COPD or asthma. A prophylactic administration may also be performed in individuals (or patients) that are likely exposed to an environment that may be highly infectious, such as healthcare professionals (nurses, doctors) working in hospitals or medical practices with SARS-CoV2-infected patients, in particular.

In one embodiment, said BKB2R-antagonist is administered to said patient at an early asymptomatic disease stage and/or at an early mild or moderate disease stage, and/or within a period of 1-10 day, preferably 1-5 days, after onset of symptomatic SARS-CoV2-infection.

The term "early asymptomatic disease stage", as used herein, is meant to refer to a stage where a patient has already tested positive for a SARS-CoV2-infection, for example in a nucleic acid amplification assay testing for virus particles, but has no symptoms of disease. The term "early mild or moderate disease stage", as used herein, is meant to refer to a disease stage, wherein a patient having a SARS-CoV2-infection shows one or several of mild or moderate symptoms, such as a mild throat pain, tiredness, fever, dry cough, loss of olfaction, headache.

Administration may occur by systemical administration or local administration. When the administration is a systemical administration, it preferably occurs by injection. In one embodiment, such injection is a subcutaneous injection. In another embodiment, the administration is by local administration which may be a topical application or an inhalative administration. A topical administration may be achieved by administration of a gel, administration of a liposomal fluid, administration of an aerosol, administration of a spray, and/or administration of a liquid, such as the administration of drops. In one embodiment, a topical administration may occur by nasal administration, oral administration or a conjunctival administration.

Inhalative administration may be achieved by a suitable administration of an aerosol or spray. An aerosol formulation typically comprises two components: a first component comprising the active pharmaceutical ingredient in neat or concentrate form, together with one or several suitable excipients, and a second component comprising a suitable propellant. The active pharmaceutical ingredient may be formulated in the first component in the form of a product concentrate which typically is a combination of the active pharmaceutical ingredient with additional suitable excipients or co-solvents required to make a stable and efficacious product. Such combination can be a solution, suspension, emulsion, semisolid or powder. The second component comprising a propellant provides the force that expels the product concentrate from a suitable container and may additionally be responsible for the delivery of the formulation in the proper form, i. e. a spray, foam, semisolid. When the propellant is liquefied gas or mixture of liquefied gases, it can also serve as the solvent or vehicle for the product concentrate. If characteristics of the active pharmaceutical ingredient are to change on dispensing, additional energy in the form of a mechanical break up system may be required.

In one embodiment, said BKB2R-antagonist is administered at a dosage of 1-100 mg daily, preferably 1-60 mg daily, more preferably 1-30 mg daily. Typically administration occurs for a duration of up to 21 consecutive days and may for example comprise administration over 1-1-5 days, 1-10 days, 1-15 days, 1-20 days and any values in between.

In one embodiment, the method(s) in accordance with the present invention is(are) performed in order to exert a protective effect on epithelial cells in said epithelial tissue against SARS-CoV2-infection, preferably without compromising an antiviral activity mounted by the patient's immune system, in particular an antiviral activity mounted in said epithelial cells.

The inventors have surprisingly found that BKB2R-antagonists do not compromise antiviral activity mounted by the patient's immune system which therefore makes BKB2R-antagonists particularly suitable for supporting a patient's immune system, particularly at an early stage of a SARS-CoV2-infection.

The inventors also surprisingly found that, in contrast to BKB2R-antagonists, corticosteroids, such as hydrocortisone, increase expression of ACE2 and TMPRSS2, which therefore seems to facilitate viral entry into cells. This finding questions the utility of corticosteroids in the context of treatment of SARS-CoV2 infections and shows the superiority of BKB2R-antagonists as a treatment option for SARS-CoV2-infections

In one embodiment, the method(s) in accordance with the present invention is(are) performed in order to reduce expression of angiotensin converting enzyme 2 (ACE2) in epithelial cells, alone or in combination with reducing expression of transmembrane protease, serine 2 (TMPRSS2) in epithelial cells, in particular IFN-gamma-induced expression of transmembrane protease, serine 2 (TMPRSS2).

In one embodiment, the method(s) in accordance with the present invention is(are) performed in order to reduce the virus titer in epithelial cells and/or to reduce the number of SARS-CoV2-virus particles formed by epithelial cells in said epithelial tissue, upon infection with SARS-CoV2.

In one embodiment, the method in accordance with the present invention results in a reduction of virus titer. Preferably, such virus titer is measured by taking a sample from the patient, and preferably quantifying the virus titer by means of a nucleic acid amplification reaction determining the number of virus particles in the sample taken. In one embodiment, the sample may be obtained from the patient using a swab. In one embodiment, the nucleic acid amplification is PCR, preferably quantitative PCR, more preferably real-time quantitative PCR. In one embodiment, prior to performing quantitative PCR on the sample, reverse transcription is performed on the sample.

In accordance with embodiments of the present invention, a reduction of virus titer, as measured, is an indication of an inhibited viral spread. This is because a reduced number of virus particles leads to a lower number of virus particles infecting further cells (e.g. epithelial cells) and thus to an impaired production of further viral particles by infected cells.

In a further aspect, the present invention also relates to a pharmaceutical composition comprising a BKB2R-antagonist, said composition being configured to allow administration of said BKB2R-antagonist by way of inhalation or topical application.

In one embodiment, said pharmaceutical composition is formulated as one of the following:
- an aerosol comprising said BKB2R-antagonist, or a composition configured to be able to form such aerosol,
- a gel comprising said BKB2R-antagonist,
- a microcapsule formulation comprising microencapsulated BKB2R-antagonist,
- a liposomal formulation comprising liposomes containing said BKB2R-antagonist,
- a spray formulation comprising said BKB2R-antagonist, or
- a drop formulation comprising said BKB2R-antagonist.

Suitable examples of aerosol and spray formulations have already been described further above.

Aerosol, spray, gel, microcapsule, liposomal and liquid ("drop") formulations of active pharmaceutical ingredients are known to a person skilled in the art and can be produced for example in accordance with Ansel's "Pharmaceutical Dosage Forms and Drug Delivery Systems", 11th edition, Wolters Kluwer, ISBN 9781496347282.

Preferably, said pharmaceutical composition is contained within a suitable container.

The present invention thus also relates to a container comprising a pharmaceutical composition comprising a BKB2R-antagonist, said composition being configured to allow administration of said BKB2R-antagonist by way of inhalation or topical application.

The present invention also relates to a dispenser device allowing the administration of a pharmaceutical composition according to the present invention, as defined herein, to a patient. In one embodiment, said dispenser device is a metered dose dispenser device. In one embodiment, said dispenser device is a multidose device. In one embodiment, said dispenser device is a multidose device allowing delivery of metered doses of pharmaceutical composition (and of the BKB2R-antagonist comprised therein). In one embodiment, said dispenser device is an inhaler device, preferably an inhaler device allowing delivery of one or several metered doses of pharmaceutical composition (and of the BKB2R-antagonist comprised therein). In one embodiment said inhaler device is breath actuated.

Having described various embodiments of the present invention, reference is now made to the following figures wherein:

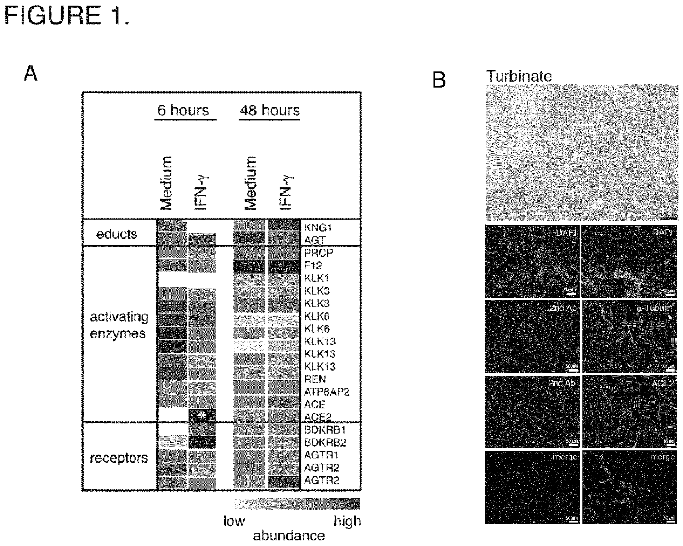

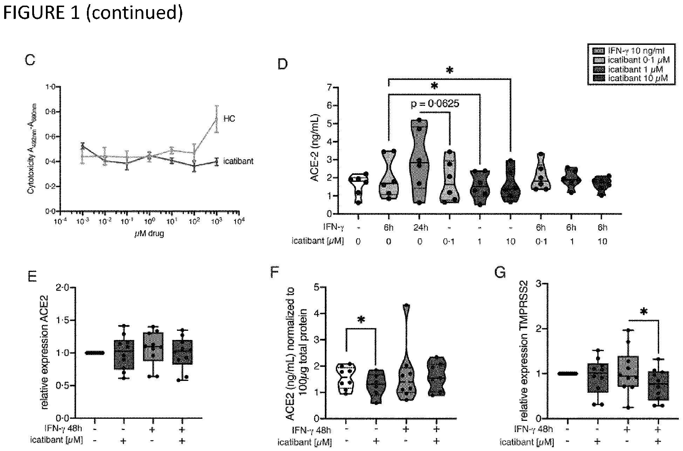

**Figure 1** shows epithelial induction of ACE2 and impact of BKB2R antagonism A) Gene expression of members of the kinin system in primary normal human bronchial epithelial cells (NHBE; n=6) after 6h or 48h of stimulation with 10 ng/ml IFN-γ. Asterisks within the grey shade plots (*) depict a gene expression fold-change >1.5 with p<0.05 of the test: IFN-γ vs medium. B) Immunohistological stainings of healthy human airway tissue from nasal turbinates for *ACE2* expression and immunofluorescence staining for DAPI, α-tubulin and ACE2. C) Lactate dehydrogenase (LDH)-assays were performed using the LDH Cytotoxicity Detection Kit PLUS to assess potential cytotoxicity of icatibant and hydrocortisone (HC) in primary NHBE. Results are depicted as mean ± s.e.m. D) ACE2 protein levels analyzed by ELISA 6h and 24h after stimulation with/without 10 ng/ml IFN and/or different concentrations of icatibant as indicated from NHBE cell lysates. E) Analysis of *ACE2* gene expression by qPCR after 48h of treatment with/without 10 ng/ml IFN-γ and/or 1 µM icatibant. F) ACE2 protein levels in NHBE cultures analyzed by ELISA from cell lysates after 48h of treatment with/without 10 ng/ml IFN-γ and or 1 µM icatibant. G) Analysis of *TMPRSS2* gene expression by qPCR after 48h of treatment with/without 10 ng/ml IFN-γ and/or 1 µM icatibant. Wilcoxon rank sum tests were used to determine significant changes in expression of the quantitative real-time PCR analysis as well as in protein levels in cell lysates. In Fig.1D, each concentration of icatibant was tested versus icatibant-untreated. In Fig.1E-G, statistical tests were performed for drug-treated versus comparable untreated condition. In addition, IFN-γ-treated was tested versus IFN-γ-untreated. Statistically significant differences were defined as P values *P<0·05, **P<0·01, and ***P<0·001.

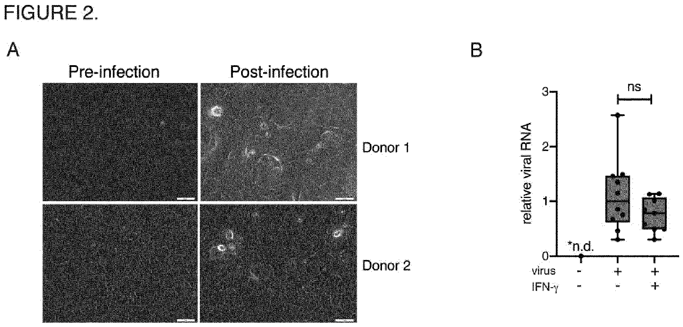

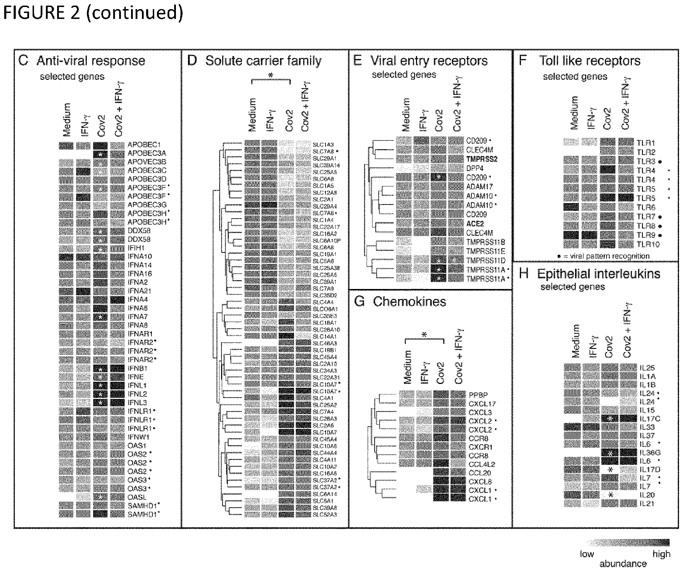

**Figure 2** shows epithelial response to SARS-CoV-2 inoculation. A) Representative images of NHBE from two donors pre- and 24h post-SARS-CoV-2-infection. B) qPCR analysis of viral N1 RNA normalized to human *ACTB* of infected primary NHBE after 24h of pre-treatment with/without 10 ng/ml IFN-γ followed by 24h of SARS-CoV-2 inoculation. Wilcoxon rank sum tests were used in Fig.2B to determine significant changes in the expression in cell lysates. IFN-γ-treated was tested versus IFN-γ-untreated. Statistically significant differences were defined as P values *P<0·05, **P<0·01, and ***P<0·001; ns= not significant; n.d.= not detected.
   Whole-genome microarray analysis of infected NHBE after 24h of pre-stimulation and subsequent 24h of SARS-CoV-2 inoculation: C) Regulation of genes involved in the immediate antiviral response of infected epithelial cells. Rhombs indicate expression of two or more isoforms that were present in the analysis. D) Gene regulation of members of the solute carrier family, proteins exhibiting membrane transport functions. Rhombs indicate expression of two or more isoforms that were present in the analysis. E) Selection of confirmed and potential viral entry receptors, rhombs indicate expression of two or more isoforms that were present in the analysis. F) Expression changes of Toll-like family members. Genes involved in virus recognition are highlighted with black dots. Rhombs indicate expression of two or more isoforms that were present in the analysis. G) Gene expression of chemokine family members, significantly regulated by SARS-CoV-2. Rhombs indicate expression of two or more isoforms that were present in the analysis. H) Gene expression of epithelium-derived interleukins. Asterisks indicates significantly regulated genes in SARS-CoV-2 compared to medium. Rhombs indicate expression of two or more isoforms that were present in the analysis.

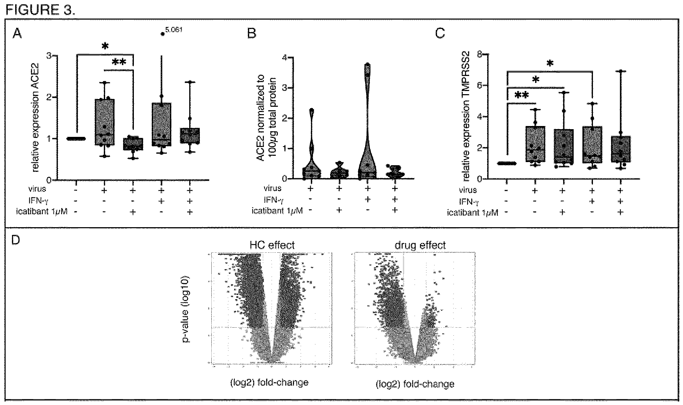

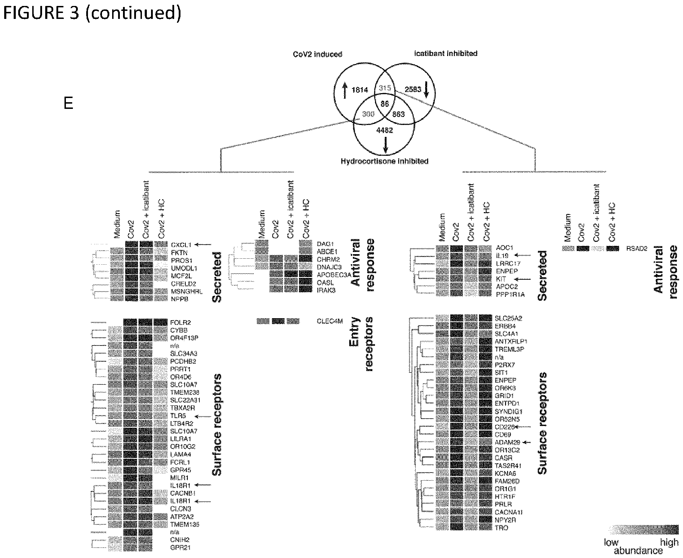

**Figure 3** shows drug-dependent gene expression changes in whole-transcriptome analysis of primary NHBE.
   A) Analysis of *ACE2* gene expression by qPCR after 24h of pre-treatment with/without 10 ng/ml IFN-y and/or 1 µM icatibant followed by 24h of SARS-CoV-2 inoculation. B) ACE2 protein levels in NHBE cultures analyzed by ELISA from cell lysates after 24h of pre-treatment with/without 10 ng/ml IFN-y and/or 1 µM icatibant followed by 24h of SARS-CoV-2 inoculation. C) Analysis of *TMPRSS2* gene expression by qPCR after 24h of pre-treatment with/without 10 ng/ml IFN-γ and/or 1 µM icatibant followed by 24h of SARS-CoV-2 inoculation. For Fig.3A-C, Wilcoxon rank sum tests were used to determine significant changes in expression of the qPCR analysis as well as in protein levels in cell lysates. Here, statistical tests were performed for icatibant-treated versus comparable untreated condition. In addition, IFN-γ-treated was tested versus IFN-γ-untreated. Statistically significant differences were defined as P values *P<0·05, **P<0·01, and ***P<0·001. D) Volcano plots revealed global gene expression changes induced by either hydrocortisone (HC) or icatibant. E) Venn diagram to depict the gene expression overlap between SARS-CoV-2-induced and HC- or icatibant-dependent inhibited genes. Rhombs indicate expression of two or more isoforms that were present in the analysis.

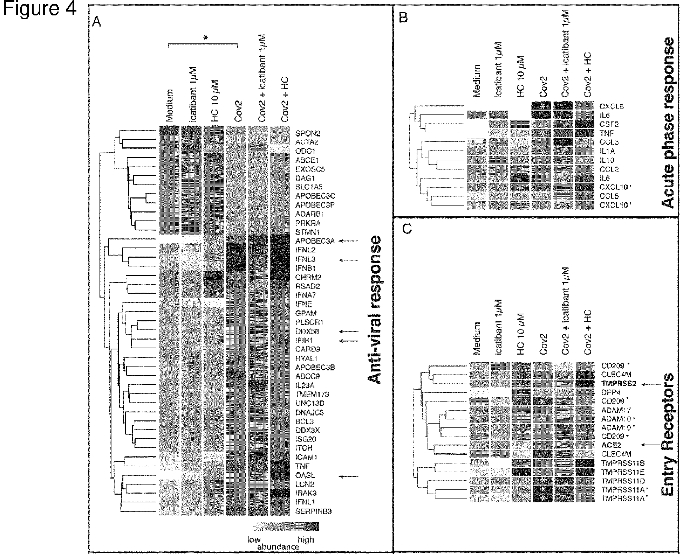

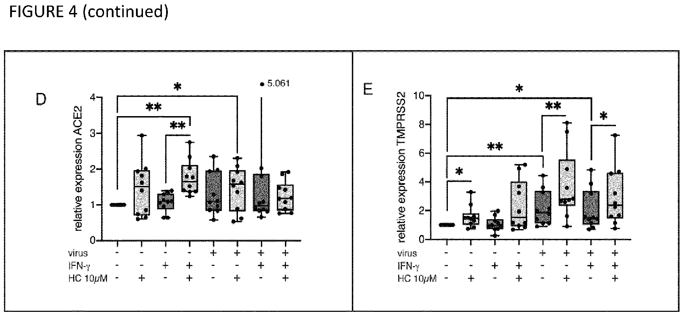

**Figure 4** shows
   A) Global gene expression analysis of genes involved in the epithelial antiviral response analyzing the effects of SARS-CoV-2-infection. Only entities with significant changes between SARS-CoV-2 and medium are depicted (gene expression fold change >1·5 with p<0·05). B) Global gene expression analysis of genes involved in the acute phase response in dependency of SARS-CoV-2-infection, icatibant or hydrocortisone (HC). C) Global gene expression analysis of known and potential virus entry receptors in dependency of SARS-CoV-2-infection, icatibant or hydrocortisone (HC). In Fig.4B&C, asterisks indicate significantly regulated genes in SARS-CoV-2 compared to medium. Rhombs indicate expression of two or more isoforms that were present in the analysis.
      Analysis of D) *ACE2* and E) *TMPRSS2* gene expression by qPCR after 24h of pre-treatment with/without 10 ng/ml IFN-γ and/or 10 µM hydrocortisone (HC) followed by 24h of SARS-CoV-2 inoculation. Asterisks indicates significantly regulated genes in SARS-CoV-2 compared to medium. Rhombs indicate expression of two or more isoforms that were present in the analysis. For Fig.4D-E, a Friedman test was performed and only if significant, statistical individual tests were carried out. Then Wilcoxon rank sum tests were performed for icatibant-treated versus comparable untreated condition. In addition, IFN-γ-treated was tested versus IFN-γ-untreated in infected and uninfected cells. Statistically significant differences were defined as P values *P<0·05, **P<0·01, and ***P<0·001.

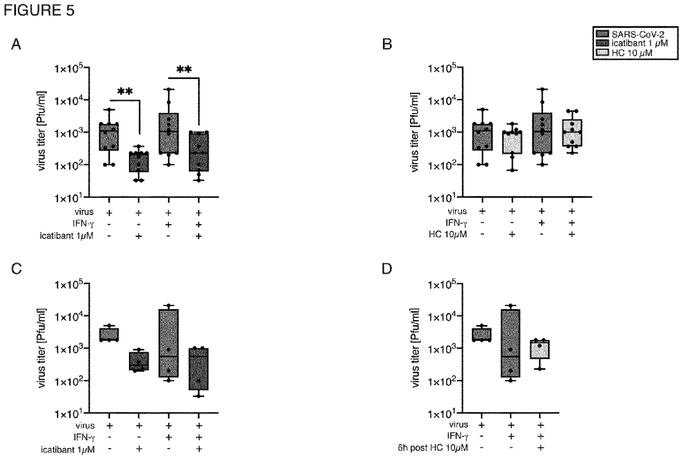

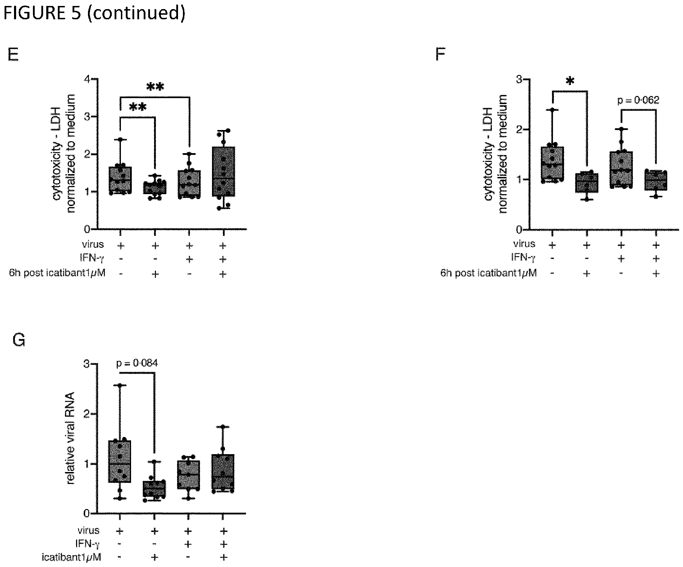

**Figure 5** shows analysis of cytopathic effects of supernatants from NHBE (n=10) after 24h of pre-treatment with/without 10 ng/ml IFN-y A) and/or 1 µM icatibant followed by 24h of SARS-CoV-2 inoculation, B) and/or 10 µM hydrocortisone (HC) followed by 24h of SARS-CoV-2 inoculation on Vero-E6 cells depicted in plaque forming units (PFU). Analysis of cytopathic effects of supernatants from NHBE (n=4) after 24h of pre-treatment with/without 10 ng/ml IFN-γ followed by 24h of SARS-CoV-2 inoculation C) and/or 1 µM icatibant added 6h post-inoculation D) and/or 10 µM hydrocortisone (HC) added 6h post-inoculation on Vero-E6 cells depicted in plaque forming units (PFU).
   E) Cytotoxicity assay determining LDH in supernatants from NHBE (n=12) after 24h of pre-treatment with/without 10 ng/ml IFN-y and/or 1 µM icatibant followed by 24h of SARS-CoV-2 inoculation. F) Cytotoxicity assay determining LDH of supernatants from NHBE after 24h of pre-treatment with/without 10 ng/ml IFN-y followed by 24h of SARS-CoV-2-inoculation with (n=6) and without (n=12) 1 µM icatibant added 6h post-inoculation. G) qPCR analysis of viral N1 RNA of infected primary NHBE (n=10), normalized to human *ACTB,* after 24h of pre-treatment with/without 10 ng/ml IFN-y and/or 1 µM icatibant followed by 24h of SARS-CoV-2 inoculation. For Fig.5A-E and G, Wilcoxon rank sum tests were used to determine significant changes. For Fig.5F, unpaired Mann-Whitney U tests were used to determine significant changes. In all subfigures, statistical tests were performed for icatibant- or HC-treated versus comparable untreated condition. In addition, IFN-γ-treated was tested versus IFN-γ-untreated. Statistically significant differences were defined as P values *P<0·05, **P<0·01, and ***P<0·001.

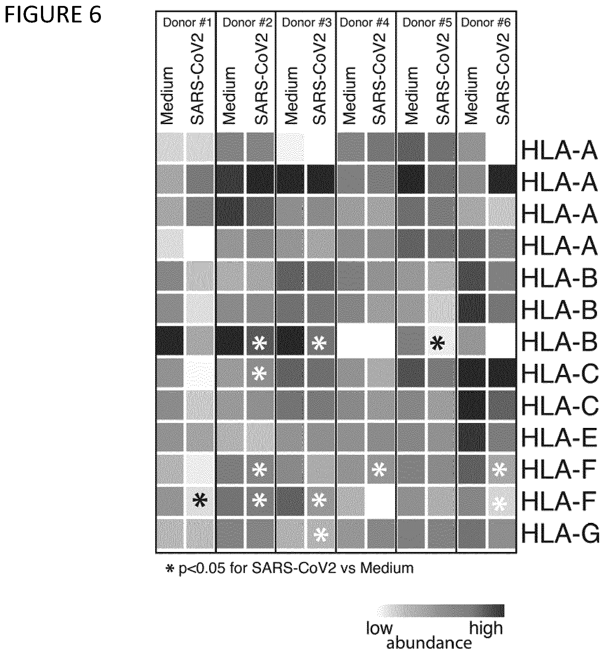

**Figure 6** shows the results of a MHC -class I gene analysis involved in the epithelial response analyzing the effects of a SARS-CoV2 infection.

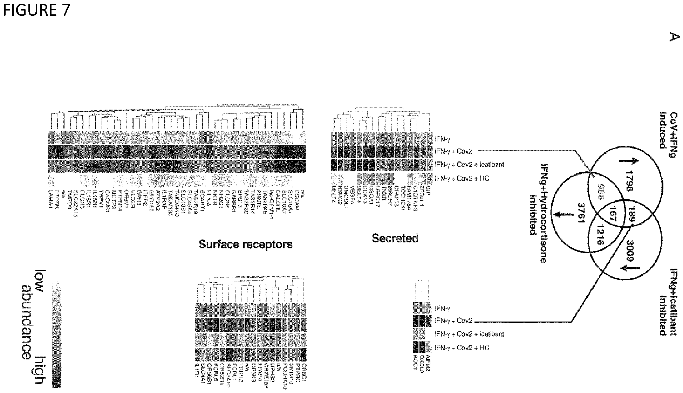

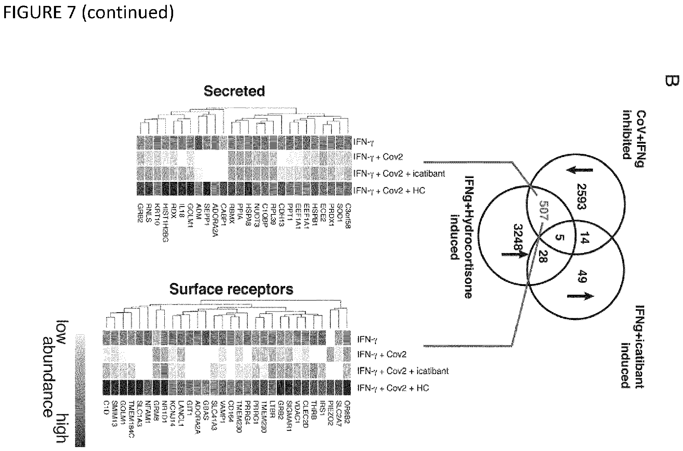

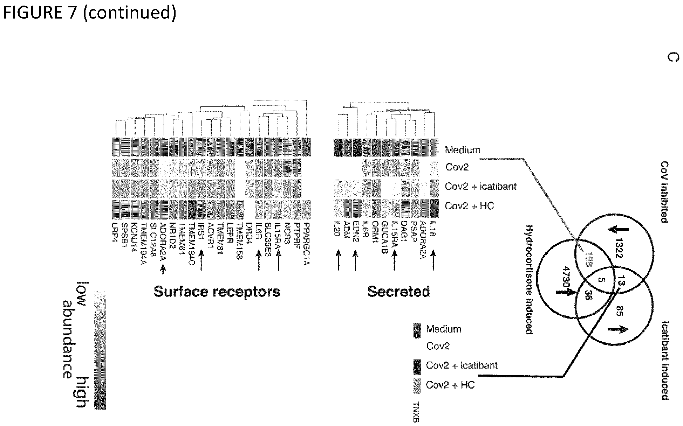

**Figure 7** A - C shows Venn diagrams to depict the gene expression overlap between SARS-CoV2-induced, SARS-CoV2-inhibited, IFN-gamma- induced, IFN-gamma-inhibited, icatibant-induced, icatibant-inhibited, hydrocortisone-induced, and hydrocortisone-inhibited genes. More specifically, Figure 7A shows secreted genes and surface receptors in the intersection of IFN-gamma/Cov2 induced genes that are otherwise downregulated when stimulated by IFN-gamma and hydrocortisone while the cross-check of IFN-gamma/Cov2 inhibited and IFN-gamma/Icatibant/hydrocortisone induced is shown in Figure 7B. Figure C shows intersection of Cov2-inhibited and Icatibant/hydrocortinsone-induced secreted genes and surface receptors without the influence of IFN-gamma.

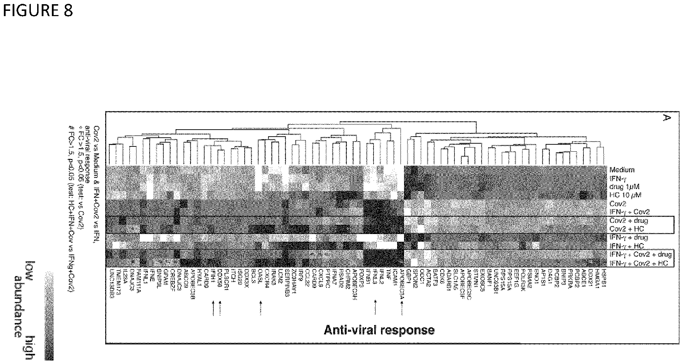

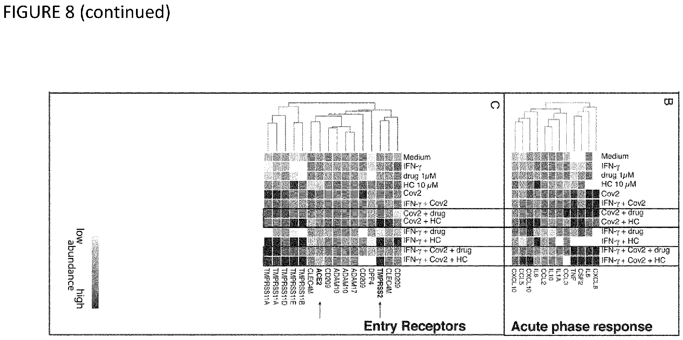

**Figure 8** Drug-dependent gene expression changes in whole- transcriptome analysis of primary NHBE (n=6) in selected gene categories. A) Global gene expression analysis of genes involved in the epithelial antiviral response comparing the effects of SARS-CoV-2 infection and IFN-γ+ SARS-CoV-2 infection. Asterisks within the grey shade plots (*) depict a gene expression fold-change >1·5 with p<0·05 of the test: SARS-CoV-2 vs medium, hashtags (#) depict a gene expression fold change >1·5 with p<0·05 of the test: HC + IFN-y + SARS-CoV-2 vs IFN-y + SARS-CoV-2. B) Global gene expression analysis of genes involved in the acute phase response in dependency of SARS-CoV-2 infection, IFN-y and HC or icatibant. C) Global gene expression analysis of known and potential virus entry receptors in dependency of SARS-CoV-2 infection, IFN-y and HC or icatibant.

Moreover, reference is made to the following examples which are given to illustrate not to limit the present invention.

### Example 1

**Methods** used in embodiments of the present invention were as follows:
**Cell culture.** Primary normal human bronchial epithelial cells (NHBE, Lonza, Walkersville, MD) from genetically independent donors were grown as monolayers in 100 % humidity and 5 % CO₂ at 37°C in serum-free defined growth media (BEGM, Lonza). NHBE were used at 80 % confluence in 6-well plates. To avoid effects induced by growth factors in the BEGM medium, cells were rested in basal medium without supplements (BEBM) for 12 hours, then stimulated with recombinant human IFN-γ at 10 ng/mL (R&D Systems) in BEBM medium for 24 hours and/or Icatibant at 1 µM or hydrocortisone at 10 µM (HC; Sigma-Aldrich, Taufkirchen, Germany), followed by further 24 hours of incubation with or without SARS-CoV-2 at the biosafety level 3 laboratory. For RNA analysis, cells were lysed in RLT buffer (Qiagen, Hilden, Germany). For Protein analysis, cells were lysed in 1x protein extraction buffer provided with the ACE2 ELISA Kit (Abcam, Cambridge, UK). Cell culture supernatant was collected for further viral and cytolytic analysis.

**Infection with SARS-CoV-2.** The SARS-CoV-2 isolate SARS-COV-2-Munich-TUM-1 was derived from patient material and amplified on Vero-E6 cells (passage 0; ATCC, Manassas, US) and used for infection experiments (passage 4) with an MOI of 0·5 (determined by Plaque assay) by adding virus stock to the BEBM culture medium after 24 hours of stimulation. The infection experiment was stopped 24 hours after inoculation.

**Statistical analysis.** Two-tailed Wilcoxon rank sum tests were used to determine significant gene expression changes analyzed by qPCR and of protein levels in ELISA analysis from cell lysates (GraphPad Prism Version 8.4.2, GraphPad Software, Inc., San Diego, CA). In Fig.5F, Mann-Whitney U tests were used. For Fig.4D and 4E, For Fig.4D-E, a Friedman test was performed and only if significant, statistical individual tests were carried out. The individual tests are specified in figure legends. Results are depicted as median with range, if not otherwise indicated in the figure legends. Statistically significant differences were defined as p-values *P<0·05, **P<0·01, and ***P<0·001.

**RNA isolation and whole genome microarray.** Total RNA was extracted using RNeasy Mini Kit (Qiagen) with on-column DNase digestion (Qiagen) for avoiding DNA contaminations. RNA quantification and quality assessments were performed by ultraviolet-visible spectrophotometry (Nanodrop Technologies, Wilmington, DE) and the RNA 6000 Nano Chip Kit with the Agilent 2100 Bioanalyzer (Agilent Technologies, Waldbronn, Germany) according to manufacturer's instructions. Microarray experiments were performed by MIAME criteria.

**Reverse transcription and quantitative real-time PCR.** Isolated total RNA was reverse transcribed using a high-capacity cDNA kit (Applied Biosystems, Foster City, CA) according to manufacturer's instructions. Real-time PCR profiles were visualized using FastStart Universal SYBR Green Mastermix (Roche, Basel, Suisse) and quantified by the ViiA 7 Real-Time PCR System (Applied Biosystems). *ACE2* and *TMPRSS2* mRNA expression was normalized to *ACTB* and *HPRT* housekeeping gene index and the relative quantification was performed using the comparative threshold cycle (2^{-ΔΔCt}) method (relative gene expression). All amplifications were carried out at least in duplicate.

**RT-qPCR-based detection of SARS-CoV-2 infection.** Total isolated RNA was subject to cDNA retranscription by SuperScript III First-Strand Synthesis SuperMix for qRT-PCR (Invitrogen) and quantified with Taqman-based SARS-CoV-2 N1 primers and probe according to CDC guidelines on a LightCycler 480 II (Roche Diagnostics, Penzberg, Germany). SARS-CoV-2 N1 RNA was normalized with relative quantification to endogenous control *ACTB* using the 1/E^{CT} formula.

**Plaque assay.** Supernatant of SARS-CoV-2 infected NHBE was titrated (1:5 dilutions of the stock) in a total volume/well of 100 µl Gibco MEM (Thermo Fisher Scientific, Waltham, US) containing 0.5 % methyl cellulose (1,500 cP, Sigma-Aldrich, St. Louis US) on 96-well plates containing 70-80 % confluent Vero-E6 cells. After 2 hours, the inoculum was discarded and cells were layered with 100 µl MEM containing 0.5 % CMC. 48 hours post inoculation, 100 µl formaldehyde was added to a final concentration of 5 % for 15 min. Supernatant was taken off, cells were washed with PBS twice and incubated with crystalviolet (Sigma-Aldrich) solution for 15 min and washed with PBS again. Plaque-forming units/ml were calculated with the formula Pfu/mL = Number of Plaques/dilution x Volume of analyzed Supernatant.

**Immunohistochemistry staining of FFPE tissue sections.** Immunohistochemistry has been performed automatically using the Bond RX^{m} system (Leica Biosystems). Antibody against ACE2 (CST Signaling, Danvers, MA, USA) was diluted 1:100, pretreated with pH6 citrate buffer.

**FFPE sections.** For lung histology, lung sections were fixed in 4 % paraformaldehyde and embedded in paraffin and sections of 4 µm thickness were produced at the routine diagnostics at the Medical School of the Technical University of Munich, Campus Biederstein.

**Immunofluorescent staining.** For immunofluorescence staining of FFPE tissue sections from human lung tissue, slides were deparaffinized and rehydrated using three washes of each: 100% ethanol for 10 min, 95% ethanol for 10 min, 70% ethanol for 10 min, 50% ethanol for 10 min, two washes of deionized water for 5 minutes each. For antigen retrieval, slides were boiled in 10 mM sodium citrate buffer (pH 6.0) using a microwave and then maintained at a sub-boiling temperature for 10 min. Slides were cooled on the bench-top for 30 min. Sections were then washed in distilled water for 5 min. For permeabilization, sections were washed twice for 10 min with 1% animal serum in PBS + 0.3% Triton X-100 (PBS-T). For the animal serum, the species of the host of the secondary antibody was used. Non-specific binding was blocked with 5% animal serum in PBS-T for 30 min at room temperature. Primary antibody staining was performed in 1% animal serum PBS-T for 2 hours at room temperature, then stored at 4°C over-night. The dilution was used as recommended by the manufacturer. Sections were washed twice with PBS for 10 min each. Secondary antibody staining was performed in 1% animal serum in PBS-T at room temperature for 2 hours at a 1:1,000 dilution. Sections were washed twice with PBS for 10 min each. Slides were mounted in anti-fade Vecta Shield Mounting Medium (Vector Laboratories, Burlingame, CA).

**Lactate Dehydrogenase Assay (LDH)** assay for assessing the cytotoxicity of virus-infected NHBE in combination with or without drug* and/or IFN-y was performed using the LDH-detecting CytoTox 96® Non-Radioactive Cytotoxicity Assay (Promega GmbH, Walldorf, Germany) according to manufacturer's instructions.

**ELISA for ACE2 levels.** Protein levels of ACE2 in NHBE cell lysates were determined using the human ACE2 ELISA Kit (Abcam, Cambridge, UK). Assays were performed according to manufacturer's instructions.

**RNA isolation and whole genome microarray.** Total RNA was extracted using RNeasy Mini Kit (Qiagen) with on-column DNase digestion (Qiagen) for avoiding DNA contaminations. RNA quantification and quality assessments were performed by ultraviolet-visible spectrophotometry (Nanodrop Technologies, Wilmington, DE) and the RNA 6000 Nano Chip Kit with the Agilent 2100 Bioanalyzer (Agilent Technologies, Waldbronn, Germany) according to manufacturer's instructions. Microarray experiments were performed by MIAME criteria.

**Microarray technology data analysis strategy.** Microarray technology data analysis was performed using the Genespring Software GX 14.9.1 (Agilent Technologies) with minimal data reduction constraints (1-5-fold change and P<0.05 cutoff) as previously described¹. Upon data import a standard baseline transformation to the median of all values was performed, including log transformation and computation of fold changes (log2(A/B)=log2(A)-log2(B)). Subsequently, a principle component analysis was conducted, which revealed a homogenous component distribution. Compromised array signals were excluded from further analysis (array spot is non-uniform if pixel noise of feature exceeds threshold or is above saturation threshold). Genes with an absolute log2 fold change larger than 1.5 and a p-value smaller than the testing level of 0.05 by using the Moderated T-Test were defined as significantly differentially expressed hits. The significantly regulated genes were summarized in entity lists (not shown). These entity lists were analyzed for overlaps using Venn diagrams. Manhattan cityblock on entities (Ward's linkage) was used to cluster changes in gene expression. GeneOntology (GO) terms "0003676", "0044212", "0000976", and "0070491" were used to discriminate transcription factors, GO terms "0007267", "0005125", "0008009", and "0005615" for identification of biomarkers, GO terms "0038023", "0004896", "0004888", and "0005887" for surface receptors, and GO terms "0009615", "0039528", "0039530", "0039639", "0051607", and "0009597" for anti-viral response.

### Example 2

### Results

### a)Epithelial induction of ACE2 and impact of BKB2R antagonism

In this example, primary human airway epithelial cells (NHBE) from ten healthy genetically independent donors were deliberately used in order to bypass cell line artifacts such as the lack of IFN-γ-induced type-i differentiation of epithelial cells. As part of the secondary, adaptive T-cell-mediated immune response to viral infections, the present inventorsinvestigated the effect of IFN-γ stimulation on the expression of genes involved in the kinin-kallikrein system in NHBE and found especially two genes to be differentially regulated: an upregulation of bradykinin receptor 2 (BKB2R) and a significantly enhanced expression of angiotensin converting enzyme 2 (ACE2) (Fig.1A). ACE2 is expressed in different human tissues, the lung being one of its localizations, which was confirmed in immunoimaging along with the localization in apical parts of nasal turbinates (Fig.iB). Compared to lower airways, the staining appeared weaker and may explain the dominance of lower airway symptoms. The finding that a type-II interferon does not only induce ACE2, but also BKB2R in NHBE, motivated the present inventors to test a selective BKB2R antagonist as therapeutic approach for the treatment of COVID-19. The present inventors therefore titrated icatibant and hydrocortisone in different doses and found that icatibant has no toxic effects in NHBE cell cultures, but even exhibits a cell-protecting effect (Fig.iC). In addition, the transcriptional regulation (Fig.1A) was confirmed on the protein level: ACE2 is upregulated in IFN-γ-stimulated cultures over time (Fig.1D). Co-treatment with icatibant, however, inhibited IFN-γ-triggered induction of ACE2. After 48 hours, this regulation disappeared on the transcriptional level (Fig.1E), but ACE2 protein levels were still significantly reduced in cells treated with icatibant at 1 µM (Fig.1F). It is known that SARS-CoV-2 requires not only the presence of ACE2, but also of the transmembrane serine protease TMPRSS2 on human lung cells in order to prime the viral S-protein for efficient binding to ACE2. IFN-γ- stimulation did not increase TMPRSS2 transcription, but icatibant significantly reduced the expression of TMPRSS2 at 1 µM in IFN-γ-treated cells (Fig.iG).

### b)Epithelial response to SARS-CoV-2 and icatibant-mediated ACE2 suppression

NHBE (n=10) were infected with patient-derived SARS-CoV-2. NHBE pre- and post-infection showed a typical microscopic picture of a balloon-like constriction of the cells after 24 hours of inoculation (Fig.2A). The successful infection was also confirmed in quantitative PCR from RNA of the inoculated cells (Fig.2B). Whole-genome microarray analyses of NHBE were performed 24 hours after SARS-CoV-2-infection with or without IFN-γ-costimulation. The transcriptome showed an immediate antiviral response of the infected epithelial cells (Fig.2C). The extraction of antiviral entities using GO terms reveals an epithelial mobilization of multiple antiviral host defense factors. In particular, induction of the spectrum of type-I and -III interferons supports effective infection of NHBE with SARS-CoV-2. Additionally, APOBEC factors are regulated upon SARS-CoV-2-infection, particularly APOBEC3A, B, C, and F. Several members of the solute carrier family were differentially regulated (Fig.2D). Selected viral entry receptors are upregulated following SARS-CoV-2-infection (Fig.2E). In addition, an up regulation of Toll-like pattern-recognition receptors was detected upon SARS-CoV-2-infection (Fig.2F). Of note, the virus downregulates the TLR9 receptor. SARS-CoV-2 inoculation significantly regulated a number of chemokines (Fig.2G) and epithelial cytokines, including IL8 (CXCL8) and CCL20 (Fig.2G), IL36G and IL17C, respectively (Fig.2H) that could attract a broad range of innate and adaptive immune cells and creative an inflammatory epithelial environment.

### c) Suppressive effects of Icatibant on airway epithelial cells

Since icatibant reduces levels of the SARS-CoV-2-entry receptor ACE2, the present inventorsattempted to intercept SARS-CoV-2-infection of NHBE. A key finding of this study is that icatibant significantly prevented induction of ACE2 expression following infection (Fig.3A). A reduction of ACE2 was also detected on protein level, independent of co-stimulation with IFN-γ (Fig.3B). However, no significant downregulation of TMPRSS2 by icatibant was detected during infection (Fig.3C).

In order to characterize this suppressive effect and estimate unwanted side effects on the lung epithelium, whole-genome transcriptomes were analyzed comparing NHBE treated with icatibant or hydrocortisone. Notably, the present inventorsobserve that treatment with icatibant has a broad suppressive effect on gene expression in epithelial cells, while hydrocortisone elicited an almost balanced relationship between suppression and induction of gene expression (Fig.3D). 300 genes were upregulated by SARS-CoV-2-infection, but downregulated by hydrocortisone (Fig.3E, left part), including the secreted factor CXCL1 and the surface receptors TLR5 and IL18R1. Similarly, 315 genes were upregulated by virus infection, but downregulated by icatibant, including the secreted pro-inflammatory factors IL19, KIT, and the surface molecules CD226 and ADAM29, both involved in adhesion and cell-matrix interactions (Fig.3E, right part). There are also 86 transcripts that were regulated by both, hydrocortisone and icatibant, however the majority are long non-coding RNAs. On the other hand, 198 genes were reduced by SARS-CoV-2-infection, but increased by hydrocortisone covering IL18, EDN2, IL20, IL15RA, IL6R and IRS1 (Fig.7C), whereas only 13 genes were significantly reduced by SARS-CoV-2-infection but increased by icatibant treatment.

### d) Hydrocortisone increases ACE2 and TMPRSS2 expression

Adverse events in treatment of COVID-19 with immunosuppressant drugs could originate from off-target effects on host antiviral defense mechanisms. Therefore, conditions were compared in respect to the antiviral epithelial response (Fig.4A). In general, changes of the antiviral epithelial response were not induced by icatibant or hydrocortisone. Antiviral APOBEC3A was even increased by both drugs (Fig.4A). Over all conditions, only RSAD2, PTPRC, CXCL9 and CARD9 were significantly decreased by icatibant (Fig.8A), while hydrocortisone significantly reduced DNAJC3, CREBZF, GPAM, BNIP3L and FAM111A. On top of these genes, no significant downregulation of antiviral response genes was found.

Further, icatibant did not have significant effects on cytokines of the acute phase response (Fig.8B). As the present inventorsobserved that icatibant inhibits SARS-CoV-2 ACE2 and TMPRSS2 expression, the present inventorsanalyzed whether icatibant and hydrocortisone also modulate accessory entry receptors or receptors identified for other corona viruses. It becomes apparent, that there are only moderate effects of the drugs on viral entry receptor, except of icatibant-mediated downregulation of CLEC4M and CD209. In contrast, hydrocortisone rather enhances receptors, like TMPRSS2, 11B and 11E proteins by hydrocortisone (Fig.4C). TMPRSS11A-E also cleave SARS-CoV-2 S-proteins. Hydrocortisone stimulation led to an upregulation of ACE2 and TMPRSS2 on the transcriptional level of uninfected cells, which was further confirmed by quantitative PCR (Fig.4D&E). ACE2 did not change in virus-infected cells upon hydrocortisone pre-treatment, but it still enhanced TMPRSS2 expression in both, uninfected and virus-infected NHBE. TMPRSS2 is upregulated by SARS-CoV-2-infection alone, and even stronger when cells were pre-treated with hydrocortisone for 24 hours (Fig.4E). The effect on TMPRSS2 is comparable between IFN-γ-co-treated and -untreated cells.

### e) Icatibant reduces viral spread of SARS-CoV-2

Finally, the effect of icatibant on viral spread was examined. NHBE (n=10) were pre-treated with or without icatibant for 24 hours and subsequently inoculated with SARS-CoV-2 for an additional 24 hours. Of note, icatibant blocked the production of infectious viral particles, in both, IFN-γ-co-stimulated and -unstimulated cells (Fig.5A). Even though hydrocortisone induced TMPRSS2 (see above), it had no adverse effect on the virus titers after 24 hours of infection (Fig.5B). In addition, a rescue approach was employed by adding icatibant (Fig.5C) or hydrocortisone (Fig.5D) to the NHBE culture 6 hours post inoculation with SARS-CoV-2. While icatibant tends to rescue the effect of infection on cells in four donors with or without pre-stimulation with IFN-γ, hydrocortisone did not. In addition, the cytopathic effect of SARS-CoV-2-infection in this setting was tested using an LDH assay. Of note, the infection-mediated cytotoxicity was significantly reduced upon pre-treatment of the cells with icatibant (Fig.5E) and upon rescue by addition of the drug six hours after inoculation (Fig.5F). Lower levels of SARS-CoV-2 N1 RNA were detected upon icatibant treatment in comparison to untreated cells after inoculation (Fig.5G). This effect was only seen in IFN-γ-naive cells. With these results, the present inventorscould show that icatibant protects lung epithelial cells and inhibits the viral spread of SARS-CoV-2.

In this example, the present inventors examined the effect of the selective bradykinin receptor 2 antagonist icatibant on the bronchial epithelium in connection with SARS-CoV-2-infection. Without wishing to be bound by any theory, the present inventors' rationale is based on the entry receptor of SARS-CoV-2, ACE2, which is an integral part of the kinin system. With the antagonism of one of the kinin system members, the present inventors speculated that either feedback mechanisms or modulated signal transduction can not only interfere with the SARS-CoV-2 spread, but also with antiviral host defense mechanisms.

### Kinin system in the airways

The kinin system is an important regulator of the vascular system, but has also been demonstrated to be important for lung fibrosis and edema. BKB2R in particular plays a major role in edema formation, which is a major complication in patients suffering from COVID-19. The role of the kinin system and the contribution of airway epithelium versus blood vessels in the airways remains partially unclear. The experiments performed in the context of the current invention shows that all bradykinin and angiotensin educts and receptors are expressed in bronchial airway epithelial cells. NHBE were exclusively used as a primary bronchial epithelial cell culture model to bypass cell line artifacts such as the lack of type I and III interferons, which are critical elements in the response of epithelial cells against viruses. The present inventors' previous studies (Zissler UM, et al. Mucosal Immunol 2016; 9(4): 917-26.) showed that epithelial cells can differentiate either into E1 or E2 cells upon priming with IFN-y and IL-4 respectively. Without wishing to be bound by any theory, the present inventors assume that cell lines incompletely commit to this polarization process. The present inventors demonstrate for the first time that the E1 phenotype goes along with increased ACE2 and that this IFN-γ-priming has an impact on the kinin system. Since IFN-y is part of the adaptive immune response, it only occurs several days after infection and without wishing to be bound by an theory, the present inventors hypothesize that IFN-γ-mediated boost of ACE2 could be responsible for the second phase of COVID-19 and contribute to the virus sepsis.

### Immunologic response

Both, SARS-CoV-2-RNA and known antiviral response genes were detected in the transcriptome of infected cell cultures. On the transcriptional level, the induction of genes involved in the immediate antiviral response of inoculated epithelial cells is supporting evidence for an effective infection of NHBE with SARS-CoV-2. A broad spectrum of type I and III interferons, which are known to induce antiviral APOBEC genes, were upregulated. In fact, APOBEC3A and B were induced, while C was significantly repressed upon SARS-CoV-2-infection. APOBEC3A and B are cytosine deaminases that the present inventors previously described to play an important role in hepatitis B virus-infection. The relevance of this differential SARS-CoV-2-induced APOBEC regulation is unclear, however the lack of APOBEC3C induction is unfortunate as it was previously shown that this enzyme inhibits coronavirus HcoV-NL63-infection of kidney cells (LLC-Mk2 cells). Its downregulation might hint to a novel evasion mechanism of SARS-CoV-2. In addition, the present inventors observed a downregulation of MHC class I molecules, in particular HLA-B and HLA-F (Fig.6).

The epithelial cells also respond with a chemotactic response such as *CXCL2, -3, CXCL17,* and *PPBP.* In addition, *IL8 (CXCL8)* and *CCL20* were previously described to be induced by SARS-CoV-2, while CXCL1, CXCL10 and CCL2, -3 and -5 were previously described for SARS-CoV-1. This CXCL1/2 response is known to recruit neutrophils. CCL20 is mainly recruiting B- and T-cells, a chemokine also induced following hepatitis C virus-infection. CXCL17 recruits dendritic cells and is important for protective immunity against herpesviridae. Viral infection also triggered *IL17C,* which may further amplify epithelial inflammation, but also *IL36G* which can promote macrophage survival. Together with virus-induced *IL6,* this effect could account for the acute cytokine storm during virus sepsis that is currently therapeutically approached by anti-IL-6R monoclonal antibody (Tocilizumab). In severe cases, pulmonary edema characterized by excessive fluid exudation has been described. SARS-CoV-2-infection changes the expression of a large number of solute carrier channels, of which 31 were up-regulated and 25 down-regulated. While the function of many of these transporter proteins is not known, it has been previously reported that some of these channel proteins can play a role in lung damage, but also in epithelial antiviral defense.

### Intervention at the kinin system compared to corticosteroids

Interventions at the level of the kinin system may curtail the epithelial hyperactivation, while maintaining critical antiviral response of the epithelium and the immune system. The knowledge about intervention specifically at the epithelial level is limited. However, corticoids have been used systemically and locally in the upper and lower airways, but are currently also used in the attempt to control the cytokine storm in the late phase of COVID-19. The present inventors therefore compared the effects of icatibant to hydrocortisone and made two major and unexpected findings:

### A) Epithelial suppression

Icatibant has broad, mainly suppressive impact on the transcription of lung epithelial genes during infection, while hydrocortisone displayed a balanced induction and repression of genes as previously reported. Thus, hydrocortisone is modulating the epithelial cells, rather than suppressing their activity with unknown outcome on COVID-19. When analyzing SARS-CoV-2-infection-regulated genes that were counter-regulated by icatibant compared to hydrocortisone, it became clear that the suppressive effects of icatibant and hydrocortisone are largely non-overlapping. It is encouraging to see for both drugs, that only a few epithelial antiviral mechanisms were compromised. Type I interferons *IFNB1* and *IFNE* and type III interferons, such as *IFNL3,* are even slightly enhanced by hydrocortisone. Both drugs enhanced the antiviral enzymes *APOBEC3A.* This is an important finding, since it is preferable to use drugs that inhibit the viral infection while maintaining the host's antiviral immune response.

The cytokine storm is an acute complication during virus sepsis of COVID-19, which is mainly but not exclusively caused by lymphocyte-derived pro-inflammatory cytokines. Since the infected epithelium appears to be the start of the SARS-CoV-2 infection, the epithelial cells also contribute to the development of the cytokine storm by attracting lymphocytes and by activation-triggered MHC and cytokine expression (*VSTM1, IL36G*).

The present inventors' data show that genes that may support the cytokine storm are also induced upon SARS-CoV-2-infection on the epithelial level, but are not significantly influenced by icatibant or hydrocortisone. By contrast, hydrocortisone recovers several pro-inflammatory cytokines (*IL18, EDN, IL20*) and surface receptors (*IL6R, IL15RA*) that are down-regulated following SARS-CoV-2-infection. Icatibant does not influence genes directly involved in the cytokine storm, but acts on genes that are relevant earlier in the initiation of immune exacerbation by activating the epithelium, for example, by direct interactions with lymphocytes via MHC class I (*CD226*), via cell adhesion mechanisms (*TRO, AOC1*), immune triggers (*IL19, KIT*), angiotensin II modification (*ENPEP*) as well as epithelial transport functions (*SLC25A2, KCNA6, CALHM4, CACNA1I*).

### B: ACE2-mediated intervention on SARS-CoV-2 infection

Icatibant but not hydrocortisone was effective in the downregulation of the virus entry receptor *ACE2* both in the presence and in the absence of IFN-γ. The expression of the entry co-receptor *TMPRSS2* was also induced upon virus infection and reduced by icatibant in the presence of IFN-γ. Interestingly, additional potential entry co-receptors, such as *TMPRSS11A, CD209, ADAM10* and *ADAM17* are up-regulated upon SARS-CoV-2, which may represent an additional strategy of the virus to amplify entry into the cells. In addition to the *ACE2* regulation, the microarray analysis shows a downregulation of *CLEC4M* by icatibant, also known as *L-SIGN,* which has been described as an additional entry receptor for SARS-CoV.

Icatibant inhibited the spread of SARS-CoV-2 in primary lung cells resulting in lower virus titers in the supernatant. The same trend was observed when icatibant was added six hours after virus inoculation. By contrast, hydrocortisone did not reduce virus titers, which is consistent with the observation that hydrocortisone even upregulated *ACE2* and *TMPRSS2.* Interestingly, hydrocortisone enhanced *ACE2* only in non-infected cells, while *TMPRSS2* was increased significantly also upon infection with SARS-CoV-2. Although these results speak against the use of corticosteroids to control the epithelial infection in COVID-19, it is important to note that hydrocortisone did not change virus titers of the cultures, presumably because the enhanced *TMPRSS2* expression is not sufficient to increase the susceptibility of the bronchial cells to SARS-CoV-2-infection. This finding indicates that a reduction in ACE2 is needed to reduce SARS-CoV-2 propagation in the airways and can be accomplished with icatibant.

In conclusion, the present inventors reveal that BKB2R-antagonizing agents, exemplified by icatibant, protect airway epithelial cells from SARS-CoV-2-infection by acting as suppressor of epithelial activation and by inhibiting viral life spread. Thus, without wishing to be bound by any theory, the present inventors believe that BKB2R-antagonizing agents, exemplified by icatibant can prevent the escalation of COVID-19 to ARDS, lung edema and virus sepsis.

## Claims

1. A bradykinin B2 receptor (BKB2R)-antagonist for use
a) in a method of preventing a SARS-CoV2-infection in a patient to progress to a disease stage **characterized by** at least one or several of acute respiratory distress syndrome (ARDS), systemic inflammatory response syndrome, severe inflammation of the lower respiratory airways, alveolar collapse and viral sepsis;
or
b) in a method of treating a SARS-CoV2-infection in a patient;
wherein said method a) or b) involves exposing epithelial tissue of a patient having or suspected of having a SARS-CoV2-infection, to said bradykinin B2 receptor (BKB2R)-antagonist.

2. The BKB2R-antagonist for use according to claim 1, wherein said epithelial tissue is **characterized by** expressing one or several viral entry proteins, preferably selected from angiotensin converting enzyme 2 (ACE2) and transmembrane protease, serine (TMPRSS) family members, such as transmembrane protease, serine 2 (TMPRSS2), wherein, more preferably, said epithelial tissue expresses both angiotensin converting enzyme 2 (ACE2) and transmembrane protease, serine 2 (TMPRSS2).

3. The BKB2R-antagonist for use according to any of claims 1 - 2, wherein said BKB2R-antagonist exerts a protective effect on epithelial cells in said epithelial tissue against SARS-CoV2-infection.

4. The BKB2R-anatagonist for use according to claim 3, wherein said protective effect of said BKB2R-antagonist on epithelial cells is achieved by said BKB2R-antagonist reducing or interfering with SARS-CoV2-entry into epithelial cells.

5. The BKB2R-antagonist for use according to claim 4, wherein reducing or interfering with SARS-CoV2-entry into the epithelial cells is achieved by said BKB2R-antagonist reducing expression of angiotensin converting enzyme 2 (ACE2), alone or in combination with reducing expression of transmembrane protease, serine 2 (TMPRSS2), in particular IFN-gamma-induced expression of transmembrane protease, serine 2 (TMPRSS2).

6. The BKB2R-antagonist for use according to any of claims 1 - 5, wherein said epithelial tissue of a patient is epithelial tissue of the respiratory tract and/or of the gastrointestinal tract, and/or is epithelial tissue primed by inflammatory conditions, such as co-existing viral or bacterial infections or interferon-driven inflammation.

7. The BKB2R-antagonist for use according to claim 6, wherein said epithelial tissue is
a) epithelial tissue of the respiratory tract and includes one or several of:
mouth epithelial tissue, nasal epithelial tissue, sinus epithelial tissue, pharyngeal epithelial tissue, laryngeal epithelial tissue, tonsillar epithelial tissue, tracheal epithelial tissue, bronchial epithelial tissue, pulmonary epithelial tissue and alveolar epithelial tissue;
or
b) epithelial tissue of the gastrointestinal tract and includes one or several of:
epithelial tissue of the mouth, epithelial tissue of the pharynx, epithelial tissue of the esophagus, epithelial tissue of the stomach, epithelial tissue of the duodenum, epithelial tissue of the jejunum, epithelial tissue of the ileum, epithelial tissue of the appendix vermiformis, epithelial tissue of the cecum, epithelial tissue of the colon, epithelial tissue of the rectum, and epithelial tissue of the anus.

8. The BKB2R-antagonist for use according to any of claims 6 - 7, wherein said epithelial tissue of a patient is
a) epithelial tissue of the respiratory tract and includes one or several types of epithelial cells of the upper respiratory tract, such as basal, ciliated, tuft or goblet cells; epithelial cells of the lower respiratory tract, such as basal, ciliated, goblet, clara cells, type-i- and -2 pneumocytes; and/or tonsillar epithelial cells;
or
b) epithelial tissue of the gastrointestinal tract and includes one or several types of epithelial cells the gastrointestinal tract, such as enterocytes, M-cells, goblet cells and tuft cells.

9. The BKB2R-antagonist for use according to any of the foregoing claims, wherein said BKB2R-antagonist is a selective BKB2R-antagonist, preferably selected from icatibant,B-9972, PHA-022121, FR173657, Fasitibant, and antibodies, or antibody fragments, against BKB2R.

10. The BKB2R-antagonist for use according to any of the foregoing claims, wherein exposing epithelial tissue of a patient having or suspected of having a SARS-CoV2-infection, to said bradykinin B2 receptor (BKB2R)-antagonist, occurs by administering said BKB2R-antagonist to said patient.

11. The BKB2R-antagonist for use according to any of the foregoing claims , wherein, in said use, said BKB2R-antagonist is administered to said patient at one or several stages selected from:
- early asymptomatic disease stage;
- early mild or moderate disease stage; or
- within a period of 1-10 days, preferably 1 - 5 days, after onset of symptomatic SARS-CoV2-infection.

12. The BKB2R-antagonist for use according to any of claims 10-11, wherein said administration is by systemical administration or local administration, wherein preferably said systemical administration is by injection, more preferably subcutaneous injection; and wherein preferably said local administration is by topical application or inhalative administration.

13. The BKB2R-antagonist for use according to claim 12, wherein said local administration is by inhalative administration, preferably by aerosol administration or spray administration; or wherein said local administration is by topical administration, preferably by administration of a gel, administration of a liposomal fluid, administration of an aerosol, administration of a spray, and/or administration of drops, wherein, more preferably, said topical administration is a nasal administration, an oral administration or a conjunctival administration.

14. The BKB2R-antagonist for use according to any of claims 11-13, wherein, during said use, said BKB2R-antagonist is administered at a dosage of 1 - 100mg daily, preferably 1 - 60mg daily, more preferably 1 - 30 mg daily; for a duration of up to 21 consecutive days.

15. The BKB2R-antagonist for use according to any of the foregoing claims, wherein said method is performed in order to exert a protective effect on epithelial cells in said epithelial tissue against SARS-CoV2-infection, preferably without compromising an antiviral activity mounted by the patient's immune system, in particular an antiviral activity mounted in said epithelial cells.

16. The BKB2R-antagonist for use according to any of the foregoing claims, wherein said method results in a reduction of virus titer.

17. A pharmaceutical composition comprising a BKB2R-antagonist, said composition being configured to allow administration of said BKB2R-antagonist by way of inhalation or topical application; wherein, preferably, said pharmaceutical composition is formulated as one of the following:
- an aerosol comprising said BKB2R-antagonist, or a composition configured to be able to form such aerosol,
- a gel comprising said BKB2R-antagonist,
- a microcapsule formulation comprising microencapsulated BKB2R-antagonist,
- a liposomal formulation comprising liposomes containing said BKB2R-antagonist,
- a spray formulation comprising said BKB2R-antagonist, or
- a drop formulation comprising said BKB2R-antagonist.
